(19) **Europäisches Patentamt European Patent Office Office européen des brevets**

(11) **EP 4 738 368 A1**

(12) **EUROPEAN PATENT APPLICATION**
published in accordance with Art. 153(4) EPC

(43) Date of publication:
06.05.2026 Bulletin 2026/19

(21) Application number: 24844860.7

(22) Date of filing: 26.07.2024

(51) International Patent Classification (IPC):
$G16B\ 20/50$ (2019.01)   $G16B\ 20/30$ (2019.01)
$G16B\ 40/10$ (2019.01)

(52) Cooperative Patent Classification (CPC):
Y02A 90/10

(86) International application number:
PCT/CN2024/107680

(87) International publication number:
WO 2025/021174 (30.01.2025 Gazette 2025/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.07.2023 CN 202310929989

(71) Applicant: Genetron Health (Beijing) Co, Ltd.
Beijing 102206 (CN)

(72) Inventors:
• CHE, Dongxue
  Beijing 102206 (CN)
• YAN, Cheng
  Beijing 102206 (CN)
• YANG, Yufei
  Beijing 102206 (CN)
• YANG, Quanyu
  Beijing 102206 (CN)
• DU, Changshi
  Beijing 102206 (CN)

(74) Representative: Lorenz Seidler Gossel Part. mbB
Widenmayerstr. 23
80538 München (DE)

(54) **APPARATUS FOR IDENTIFYING TRUE MUTATION OR SEQUENCING NOISE IN CTDNA SEQUENCING DATA, COMPUTER-READABLE STORAGE MEDIUM, AND USE**

(57)    The disclosure discloses an apparatus, a computer-readable storage medium, and applications for identifying true mutations or sequencing noise in ctDNA sequencing data. This disclosure constructs noise levels at loci using a dataset from healthy individuals and determines true mutation signals by statistically testing the differences between the estimated noise levels and the observed mutation frequencies in actual tumor samples. Compared to the prior art, which employs a unified positive threshold for all loci, the present disclosure utilizes a positive threshold specific to each locus, making it more sensitive in distinguishing low-frequency noise or true mutation signals. Moreover, it does not require data from matched normal control samples of tumor patients, thereby reducing experimental and sequencing costs. It can be applied in the preparation of products for screening or auxiliary screening of cancer patients, as well as products for personalized cancer therapy.

**Description**

**TECHNICAL FIELD**

The disclosure relates to an apparatus, a computer-readable storage medium, and uses for identifying true mutations or sequencing noise in ctDNA sequencing data within the field of bioinformatics.

**BACKGROUND**

Liquid biopsy technology, as a branch of in vitro diagnostics, enables the analysis and diagnosis of diseases such as cancer by sampling cerebrospinal fluid, saliva, blood, urine, etc., and can, to a certain extent, circumvent the influence of tissue heterogeneity on diseases. Currently, the detection of circulating tumor DNA (ctDNA) in blood represents the primary research direction within liquid biopsy. Circulating tumor DNA (ctDNA) refers to DNA fragments released by tumor cells that carry tumor mutation information. With the advancement of high-throughput technologies, the objective of ctDNA NGS data detection is to analyze specific mutated genes within tumor cells and subsequently provide personalized therapy plans for cancer patients. As sequencing throughput continues to increase and data volumes grow exponentially, this simultaneously poses significant challenges to mutation detection techniques. The difficulty in detecting ctDNA data lies in the often low frequency of tumor mutations, making it crucial to distinguish between low-frequency tumor mutations and sequencing noise amidst the vast data throughput. Existing detection technologies assume uniform noise levels across loci and employ a unified threshold standard to differentiate mutations from noise, which may reduce sensitivity or specificity at certain loci and even result in false-negative or false-positive loci. Moreover, current mutation detection techniques require the provision of normal control samples paired with cancer patients, directly leading to increased costs.

**SUMMARY**

The present disclosure provides an apparatus and method for detecting noise levels in ctDNA sequencing data, primarily utilizing locus-based noise levels to detect mutations. This disclosure constructs background noise levels at loci using datasets from healthy individuals and determines true mutation signals through statistical tests comparing the estimated noise levels with the observed mutation frequencies in actual tumor samples. Compared to existing technologies that employ a uniform positive cutoff value across all loci, the present disclosure utilizes locus-specific positive cutoff values, making it more sensitive in distinguishing low-frequency noise/mutation signals. The technology of the present disclosure can detect SNVs and InDels in ctDNA data from single tumor samples without requiring data from paired normal samples of cancer patients, thereby reducing experimental and sequencing costs.

Technical Problem

The technical problem to be addressed by the present disclosure is how to accurately identify or detect whether the mutations in ctDNA sequencing data are true mutations or sequencing noise.

Technical Solution

In order to address the aforementioned technical problems, the present disclosure first provides an apparatus for detecting noise levels in ctDNA sequencing data, which may comprise the following modules:

A1) ctDNA Sequencing Data Processing Module: configured to obtain or receive ctDNA sequencing data from healthy subjects, perform quality control, and align the data with the human reference genome to generate alignment files;

A2) Mutation Frequency Detection Module: configured to obtain the sequencing depth at each locus, the number of reads supporting single-nucleotide variant at each locus, and the number of reads supporting insertion or deletion at each locus in the ctDNA sequencing data based on the alignment files. For each locus, the module calculates: (i) the single-nucleotide variant frequency based on the sequencing depth and the number of reads supporting single-nucleotide variant, and (ii) the insertion or deletion frequency based on the sequencing depth and the number of reads supporting insertion or deletion.

A3) Single-Nucleotide Variant Noise Level Model Construction Module: configured to obtain the single-nucleotide variant noise level at each locus based on the grouping information of single-nucleotide variant feature at each locus and the single-nucleotide variant frequency at each locus; wherein the grouping information of single-nucleotide variant feature at each locus includes the trinucleotide sequence context and the alignment feature at each locus;

A4) Insertion or Deletion Noise Level Model Construction Module: configured to obtain the insertion or deletion noise level at each locus based on the grouping information of insertion or deletion feature at each locus and the insertion or deletion frequency at each locus; wherein the grouping information of insertion or deletion feature at each locus includes the repetitive sequence element information and the segmental duplication information at each locus;

A5) Noise Level Output Module: configured to output the noise levels of the ctDNA sequencing data, which include the single-nucleotide variant (SNV) noise level at each locus and/or the insertion or deletion (InDel) noise level at each locus.

The aforementioned loci may refer to a single nucleotide, specifically those included within the range of a sequencing panel. The trinucleotide sequence context at each locus consists of the nucleotide at that locus on the reference genome and one nucleotide each from its upstream and downstream, forming a three-nucleotide sequence. The trinucleotide sequence context at each locus can be obtained from the human reference genome file. The alignment feature at each locus may be a unique alignment or multiple alignments, which can be obtained from the Mappability file of the human reference genome. The repetitive sequence element information at each locus indicates whether the locus belongs to a repetitive sequence region and, if so, the specific type of repetitive region to which it belongs. This information can be obtained from the repetitive sequence element data file of the human genome. The segmental duplication information at each locus indicates whether the locus belongs to a large segmental duplication region, which can be obtained from the segmental duplication data file of the human genome.

In the aforementioned apparatus, the Single-Nucleotide Variant Noise Level Model Construction Module (A3) may include the following modules:

A3-1) Single-Nucleotide Variant Characteristic Integration and Grouping Module: configured to group loci that share the same trinucleotide sequence context and the same alignment feature together, thereby forming groups with identical SNV feature.

A3-2) Single-Nucleotide Variant Prior Background Noise Level Acquisition Module: configured to calculate the average single-nucleotide variant frequency of each group with identical SNV feature using Formula 1, based on the mutation frequencies of all loci within the group with identical SNV feature and the group with identical SNV feature described in A3-1.:

$$\lambda_{tm} = \frac{\sum \mu_{ptm}}{n} \qquad \text{Formula 1,}$$

in Formula 1, $\lambda_{tm}$ represents the average single-nucleotide variant frequency of all loci within the group with identical SNV feature, $\mu_{ptm}$ represents the single-nucleotide variant frequency at each locus within the group with identical SNV feature described in A3-1), and n represents the number of loci within the group with identical SNV feature described in A3-1);

A3-3) Single-Nucleotide Variant Noise Level Acquisition Module: configured to obtain the single-nucleotide variant noise level at each locus using Formulas 2 and 3, based on the average single-nucleotide variant frequency of the group with identical SNV feature to which each locus belongs and the single-nucleotide variant frequency at each locus:

$$\varepsilon_p = \omega_1 \lambda_{tm} + (1 - \omega_1) \mu_p \qquad \text{Formula 2}$$

$$\omega_1 = \frac{\lambda_{tm}}{\lambda_{tm} + \mu_p} \qquad \text{Formula 3,}$$

in Formulas 2 and 3, p represents a certain locus within the range of the sequencing panel, $\lambda_{tm}$ represents the average single-nucleotide variant frequency of all loci within the group with identical SNV feature to which locus p belongs, $\mu_p$ represents the single-nucleotide variant frequency at locus p, $\omega_1$ represents the weight, and $\varepsilon_p$ represents the single-nucleotide variant noise level at locus p.

In the aforementioned apparatus, the Mappability file may be sourced from the UCSC website (URL: https://genome.ucsc.edu/cgi-bin/hgTables/hgsid=1510554375_HANSVqcS3xxfWO2Ui7wY z8FFIlzA, reference genome version: Hg19; 100bp reads length alignment version).

In the aforementioned apparatus, the Insertion or Deletion Noise Level Model Construction Module (A4) may include the following modules:

A4-1) Insertion or Deletion Characteristic Integration and Grouping Module: configured to group loci that share the same repetitive sequence element information and the same segmental duplication information together, thereby forming groups with identical InDel feature.

A4-2) Insertion or Deletion Prior Background Noise Level Acquisition Module: configured to calculate the average insertion or deletion frequency of each group with identical InDel feature using Formula 4, based on the insertion or deletion frequency of all loci within the group with identical InDel feature and the group with identical InDel feature described in A4-1):

$$\lambda_{rsl} = \frac{\sum \mu_{prsl}}{m} \qquad \text{Formula 4,}$$

in Formula 4, $\lambda_{rsl}$ represents the average insertion or deletion frequency of all loci within the group with identical InDel feature, $\mu_{prsl}$ represents the insertion or deletion frequency at each locus within the group with identical InDel feature described in A4-1), and m represents the number of loci within the group with identical InDel feature described in A4-1);

A4-3) Insertion or Deletion Noise Level Acquisition Module: configured to obtain the insertion or deletion noise level at each locus using Formulas 5 and 6, based on the average insertion or deletion frequency of the group with identical InDel feature to which each locus belongs and the insertion or deletion frequency at each locus:

$$\omega_2 = \frac{\lambda_{rsl}}{\lambda_{rsl} + \mu_{pl}} \qquad \text{Formula 5}$$

$$\varepsilon_{pl} = \omega_2 \lambda_{rsl} + (1 - \omega_2)\, \mu_{pl} \quad \text{Formula 6,}$$

in Formulas 5 and 6, p represents a certain locus within the range of the sequencing panel, $\omega_2$ represents the weight, $\lambda_{rsl}$ represents the average insertion or deletion frequency of all loci within the group with identical InDel feature to which locus p belongs, $\mu_{pl}$ represents the insertion or deletion frequency at locus p, and $\varepsilon_{pl}$ represents the insertion or deletion noise level at locus p.

In the aforementioned apparatus, the repetitive sequence element information includes eight types: DNA repeat elements, long insert repeat elements, short insert repeat elements, low complexity region repeat elements, simple repeat region repeat elements, long segment repeat region elements, non-repetitive regions, and other repetitive elements. The segmental duplication information includes two types: located in non-large segmental duplication regions and located in large segmental duplication regions. The repetitive sequence element data file (Repeats file) of the human genome may be sourced from the UCSC website (URL: https://genome.ucsc.edu/cgi-bin/hgTables, reference genome version: Hg19, select RepeatMasker). The segmental duplication data file (Segmental Duplication file) of the human genome may be sourced from the UCSC website (URL: https://genome.ucsc.edu/cgi-bin/hgTables, reference genome version: Hg19, select Segmental Dups).

The aforementioned sequencing data may refer to the sequencing data corresponding to the panel of the sequencing product.

In order to address the aforementioned technical problems, this disclosure also provides an apparatus for identifying whether the mutations in ctDNA sequencing data are true mutations or sequencing noise. The apparatus may include the following modules:

C1) ctDNA Sequencing Data Quality Control and Alignment Module: configured to obtain or receive ctDNA sequencing data from the sample to be tested, perform quality control, and align the data with the human reference genome to generate alignment files;

C2) ctDNA Sequencing Data Mutation Analysis Module: configured to obtain the sequencing depth, the number of reads supporting mutation , and the mutation frequency at each locus based on the alignment files, thereby obtaining mutations in the ctDNA sequencing data of the sample to be tested;

C3) Statistical Testing Module: configured to determine whether the mutations are true mutations or sequencing noise using statistical hypothesis testing methods based on the mutations and noise levels. The noise levels can be obtained using the apparatus described above.

In the aforementioned apparatus, the mutations can be SNVs and/or InDels.

In the aforementioned apparatus, the noise levels can be SNV noise levels and/or InDel noise levels.

In the aforementioned apparatus, the statistical hypothesis testing can use Formulas 7 and 8 to calculate the statistical significance level of the hypothesis test:

$$\lambda_p = d_p \cdot \delta_p \qquad \text{Formula 7;}$$

$$P(k_p, \lambda_p) = 1 - \sum_{j=0}^{k_p} \frac{e^{-\lambda_p} \lambda_p^j}{j!} \qquad \text{Formula 8;}$$

in Formulas 7 and 8: p represents a certain locus within the range of sequencing panel; $\delta_p$ is the noise level (SNV noise level, InDel noise level) at locus p; $d_p$ is the sequencing depth at locus p; $\lambda_p$ is the expected number of reads supporting noise mutation observed at locus p; $k_p$ is the number of reads supporting mutation ( SNV, InDel) at locus p in the sample to be tested; j represents the value of the number of reads supporting mutation, $j \in (0, k_p)$; $P(k_p, \lambda_p)$ represents the statistical P-value for $k_p$ being greater than or equal to $\lambda_p$.

In order to address the aforementioned technical problems, this disclosure also provides a method for detecting noise levels in ctDNA sequencing data, which may include the following steps:

B1) Processing ctDNA sequencing data to obtain ctDNA sequencing data from healthy subjects, and align the data with the human reference genome after quality control to generate the alignment files;

B2) Performing mutation frequency detection: based on the alignment files, to obtain the sequencing depth, the number of reads supporting single-nucleotide variant, and the number of reads supporting insertion or deletion - at each locus in the ctDNA sequencing data. At each locus, calculate the single-nucleotide variant frequency based on the sequencing depth and the number of reads supporting single-nucleotide variant, and calculate the insertion or deletion mutation frequency based on the sequencing depth and the number of reads supporting insertion or deletion ;

B3) Constructing single-nucleotide variant noise level model to obtain the single-nucleotide variant noise level at each locus based on the grouping information of SNV feature at each locus and the single-nucleotide variant frequency at each locus; wherein the grouping information of SNV feature at each locus includes the trinucleotide sequence context and the alignment feature;

B4) Constructing insertion or deletion noise level model to obtain the insertion or deletion noise level at each locus based on the grouping information of InDel feature at each locus and the insertion or deletion frequency at each locus; wherein the grouping information of InDel feature at each locus includes the repetitive sequence element information and the segmental duplication information;

B5) Outputting the noise levels in the ctDNA sequencing data; which include the single-nucleotide variant (SNV) noise level at each locus and/or the insertion or deletion mutation (Indel) noise level at each locus.

The aforementioned locus can be a single nucleotide, specifically a locus included within the range of sequencing panel. The trinucleotide sequence context at each locus includes three nucleotides consists of the nucleotide at that locus on the reference genome and one nucleotide each from its upstream and downstream. The trinucleotide sequence context at each locus can be obtained from the human reference genome file. The alignment feature at each locus can be a unique alignment or multiple alignments, which can be obtained from the Mappability file of the human reference genome. The repetitive sequence element information at each locus indicates whether the locus belongs to a repetitive sequence region and, if so, the specific type of repetitive region to which it belongs. The repetitive sequence element information at each locus can be obtained from the repetitive sequence element data file of the human genome. The segmental duplication information at each locus indicates whether the locus belongs to a large segment duplication region, which can be obtained from the segmental duplication data file of the human genome.

In the aforementioned method, the construction of the single-nucleotide variant noise level model in B3) may include the following steps:

B3-1) Integrating and grouping single-nucleotide variant groups based on the trinucleotide sequence context and alignment feature at each locus, to group loci with the same trinucleotide sequence context and the same alignments feature together, thereby forming groups with identical SNV feature;

B3-2) Acquiring the prior background noise level of single-nucleotide variant to calculate the average single-nucleotide variant frequency of the group with identical SNV feature using Formula 1, based on the single-nucleotide variant frequency of all loci within the group with identical SNV feature and the group with identical SNV feature described in B3-1):

$$\lambda_{tm} = \frac{\sum \mu_{ptm}}{n} \qquad \text{Formula 1;}$$

in Formula 1: $\lambda_{tm}$ represents the average single-nucleotide variant frequency of all loci within the group with identical SNV feature; $\mu_{ptm}$ represents the single-nucleotide variant frequency at each locus within the group with identical SNV feature described in B3-1); n represents the number of loci within the group with identical SNV feature described in B3-1);

B3-3) Acquiring the single-nucleotide variants noise level at each locus using Formulas 2 and 3, based on the average single-nucleotide variant frequency of the group with identical SNV feature to which each locus belongs and the single-nucleotide variant frequency at each locus:

$$\varepsilon_p = \omega_1 \lambda_{tm} + (1 - \omega_1) \mu_p \qquad \text{Formula 2;}$$

$$\omega_1 = \frac{\lambda_{tm}}{\lambda_{tm} + \mu_p} \qquad \text{Formula 3;}$$

in Formulas 2 and 3: p represents a certain locus within the range of sequencing panel; $\lambda_{tm}$ represents the average single-nucleotide variant frequency of all loci within the group with identical SNV feature to which locus p belongs; $\mu_p$ represents the single-nucleotide variant frequency at locus p; $\omega_1$ represents the weight; $\varepsilon_p$ represents the single-nucleotide variant noise level at locus p.

In the aforementioned method, the Mappability file can be sourced from the UCSC website (URL: https://genome.ucsc.edu/cgi-bin/hgTables/hgsid=1510554375_HANSVqcS3xxfWO2Ui7wY z8FFIlzA, reference genome version: Hg19; 100bp reads length alignment version).

In the aforementioned method, the construction of the insertion or deletion noise level model in B4) may include the following steps:

B4-1) Integrating and grouping insertion or deletion groups based on the repetitive sequence element information and the segmental duplication information at each locus, to group loci with the same repetitive sequence element information and the same segmental duplication information together, thereby forming groups with identical InDel feature;

B4-2) Acquiring the prior background noise level of insertion or deletion to calculate the average insertion or deletion frequency of the group with identical InDel feature using Formula 4, based on the insertion or deletion frequency at each locus of the group with identical InDel feature and the group with identical InDel feature described in B4-1):

$$\lambda_{rsl} = \frac{\sum \mu_{prsl}}{m} \qquad \text{Formula 4;}$$

in Formula 4: $\lambda_{rsl}$ represents the average insertion or deletion frequency of all loci within the group with identical InDel feature; $\mu_{prsl}$ represents the insertion or deletion frequency at each locus within the group with identical InDel feature described in B4-1); m represents the number of loci within the group with identical InDel feature described in B4-1);

B4-3) Acquiring the insertion or deletion noise level at each locus using Formulas 5 and 6, based on the average insertion or deletion frequency of the group with identical InDel feature to which each locus belongs and the insertion or deletion frequency at each locus:

$$\omega_2 = \frac{\lambda_{rsl}}{\lambda_{rsl} + \mu_{pl}} \qquad \text{Formula 5;}$$

$$\varepsilon_{pl} = \omega_2 \lambda_{rsl} + (1 - \omega_2) \mu_{pl} \qquad \text{Formula 6;}$$

in Formulas 5 and 6: p represents a certain locus within the range of sequencing panel; $\omega_2$ represents the weight; $\lambda_{rsl}$ represents the average insertion or deletion frequency of all loci within the group with identical InDel feature to which

locus p belongs; $\mu_{pI}$ represents the insertion or deletion frequency at locus p; $\varepsilon_{pI}$ represents the insertion or deletion noise level at locus p.

In the aforementioned method, the repetitive sequence element information includes eight types: DNA repeat elements, long insert repeat elements, short insert repeat elements, low-complexity region repeat elements, simple repeat region repeat elements, long segment repeat region elements, non-repeat regions, and other repeat elements. The segmental duplication information includes two types: located in non-large segment duplication regions and located in large segment duplication regions. The repetitive sequence element data file (Repeats file) of the human genome can be sourced from the UCSC website (URL: https://genome.ucsc.edu/cgi-bin/hgTables, reference genome version: Hg19, select RepeatMasker). The chromosomal segment duplication data file (Segmental Duplication file) of the human genome can be sourced from the UCSC website (URL: https://genome.ucsc.edu/cgi-bin/hgTables, reference genome version: Hg19, select Segmental Dups).

The aforementioned sequencing data can be the sequencing data corresponding to the panel of the sequencing product.

In order to address the aforementioned technical problems, this disclosure also provides a method for identifying whether mutations in ctDNA sequencing data are true mutations or sequencing noise, which may include the following steps:

D1) Acquiring the ctDNA sequencing data from the sample to be tested, performing quality control and aligning the data with the human reference genome to generate alignment files;

D2) Performing mutation analysis of ctDNA sequencing data to obtain the sequencing depth, the number of reads supporting mutation, and the mutation frequency at each locus based on the alignment files, thereby obtaining the mutations in the ctDNA sequencing data of the sample to be tested;

D3) Performing statistical testing analysis to determine whether the mutations are true mutations or sequencing noise using a statistical hypothesis testing method based on the mutations and noise levels; the noise levels can be obtained using the method described above.

In the aforementioned method, the mutations can be SNVs and/or InDels.

In the aforementioned method, the noise levels can be SNV noise levels and/or InDel noise levels.

In the aforementioned method, the statistical hypothesis testing can use Formulas 7 and 8 to calculate the statistical significance level of the hypothesis test:

$$\lambda_p = d_p \cdot \delta_p \qquad \text{Formula 7;}$$

$$P(k_p, \lambda_p) = 1 - \sum_{j=0}^{k_p} \frac{e^{-\lambda_p} \lambda_p^j}{j!} \qquad \text{Formula 8;}$$

in Formulas 7 and 8: p represents a certain locus within the sequencing panel range; $\delta_p$ is the noise level (SNV noise level, InDel noise level) at locus p; $d_p$ is the sequencing depth at locus p; $\lambda_p$ is the expected number of reads supporting noise mutation observed at locus p; $k_p$ is the number of reads supporting mutation (SNV, InDel) at locus p in the sample to be tested; j represents the value of the number of reads supporting mutation, $j \in (0, k_p)$; $P(k_p, \lambda_p)$ represents the statistical P-value for $k_p$ being greater than or equal to $\lambda_p$.

In order to address the aforementioned technical problems, this disclosure also provides a computer-readable storage medium storing a computer program, which can be a computer-readable storage medium for detecting noise levels in ctDNA sequencing data or a computer-readable storage medium for identifying true mutations or sequencing noise in ctDNA sequencing data. The computer program of the computer-readable storage medium for detecting noise levels in ctDNA sequencing data or the computer-readable storage medium for identifying true mutations or sequencing noise in ctDNA sequencing data can enable a computer to execute the steps of the method described above or run the modules of the apparatus described above.

In order to address the aforementioned technical problems, this disclosure also provides a computer apparatus, including a memory, a processor, and a computer program stored on the memory. The processor can execute the computer program to realize the steps B1)-B5) or D1)-D3) of the method described above.

In order to address the aforementioned technical problems, this disclosure also provides a computer program product, including a computer program. When executed by a processor, the computer program can realize the steps of the method described above.

Any of the following uses of the aforementioned apparatus also fall within the scope of protection of this disclosure:

E1) use in preparing products for screening or auxiliary screening of cancer patients;
E2) use in developing or preparing products for personalized cancer therapy.

Any of the following uses of the aforementioned method also fall within the scope of protection of this disclosure:

E1) use in preparing products for screening or auxiliary screening of cancer patients;
E2) use in developing or preparing products for personalized cancer therapy.

Any of the following uses of the aforementioned computer-readable storage medium also fall within the scope of protection of this disclosure:

E1) use in preparing products for screening or auxiliary screening of cancer patients;
E2) use in developing or preparing products for personalized cancer therapy.

Any of the following uses of the aforementioned computer apparatus also fall within the scope of protection of this disclosure:

E1) use in preparing products for screening or auxiliary screening of cancer patients;
E2) use in developing or preparing products for personalized cancer therapy.

Any of the following uses of the aforementioned computer program product also fall within the scope of protection of this disclosure:

E1) use in preparing products for screening or auxiliary screening of cancer patients;
E2) use in developing or preparing products for personalized cancer therapy.

The aforementioned computer program product can be a software product that mainly achieves its solution through a computer program.

The aforementioned computer-readable storage medium refers to a carrier for storing data, which can be magnetic tapes, magnetic disks, floppy disks, optical disks, magneto-optical disks, ROMs, PROMs, VCDs, DVDs, hard disks, flash drives, USB drives, CF cards, SD cards, MMC cards, SM cards, Memory Sticks, or xD cards, etc.

In response to the aforementioned problems, the objective of this disclosure is to provide a method for detecting mutations based on locus-specific noise levels.

The technology of this disclosure does not require data from matched normal control samples of tumor patients for detecting SNVs and InDels in ctDNA data from single tumor samples, which reduces experimental and sequencing cost investments.

Beneficial Effects

1. For detection of single-nucleotide variants (SNVs) and/or insertions and deletions (InDels) in sequencing data of ctDNA samples, this disclosure does not need to provide matched normal control samples, thereby reducing cost investment.

2. This disclosure constructs the background noise level for each locus in panel data using a dataset from healthy donors, and determines true mutation signals by statistically testing the differences between the estimated noise level and the mutation frequency observed in tumor samples. Compared with the prior art, which uses a unified positive threshold for all loci, this disclosure uses locus-specific positive thresholds, providing higher sensitivity for distinguishing low-frequency noise from true mutation signals.

3. This disclosure simulates locus-specific noise levels for different loci within the sequencing data, which offers significant advantages in evaluating the limit of detection (LOD) for each locus.

4. This disclosure is simple to operate, consumes minimal computational resources, and offers high processing speed. Since it uses a healthy individual dataset to construct a noise level model, when analyzing real tumor samples, if sequencing panel, experimental conditions, etc., remain unchanged, there is no need to reconstruct the model. Statistical tests can be directly performed based on the noise level and the observed mutation frequency.

**BRIEF DESCRIPTION OF THE DRAWINGS**

FIG. 1 is a schematic diagram for constructing a noise level model used ctDNA sequencing data.

FIG. 2 is a comparison of mutation site detection results by different detection methods, where AF represents the mutation frequency.

**DETAILED DESCRIPTION**

The present disclosure will be further described in detail below in conjunction with specific embodiments. The provided examples are solely intended to illustrate the disclosure and not to limit its scope. The examples presented here can serve as a guide for professionals in this technical field to make further improvements and do not in any way constitute a restriction on the present disclosure.

Unless otherwise specified, the experimental methods in the following examples are all conventional methods, carried out in accordance with the techniques or conditions described in the literature within this field or as per the product instructions. Unless otherwise stated, the materials, reagents, etc., used in the following examples can all be obtained through commercial channels.

Example 1: Construction of a noise level model for loci based on ctDNA sequencing data from healthy individuals

This disclosure constructs a noise level model for loci based on ctDNA sequencing data from 40 healthy individual samples collected by Genetron Health(Beijing) Co., Ltd. (with no abnormalities in the annual blood test reports of the samples). Using the Onco Sonar product (CT_DNA_170 Solid Tumor Gene Mutation Detection Kit (Reversible Terminator Sequencing Method), Genetron Health(Beijing) Co., Ltd., product catalog number RSN113), in accordance with the standard experimental procedures, the original ctDNA sequencing data of 40 healthy individuals without known cancer were obtained through steps such as DNA extraction, library construction, probe capture, and sequencing. This example includes steps such as pre - processing of the original data, obtaining mutation frequencies, determining mutation grouping characteristics, and constructing a noise level model.

1. Pre - processing of original data

The original sequencing data is in FASTQ format. Standard data analysis procedures such as quality control (including data volume, sequencing depth before/after deduplication, Q30, and capture efficiency, etc.) and alignment are performed on the original ctDNA sequencing data of the 40 healthy individual samples. The quality control and alignment software are Trimmomatic (v0.33, http://www.usadellab.org/cms/index.php?page=trimmomatic) and BWA (v0.7.10 - r789, https://bio - bwa.sourceforge.net/), respectively.

The quality control software Trimmomatic requires parameter settings, including providing the adapter sequence used for sequencing; setting the sliding window trimming size to 4 bp, with the average base quality score within the sliding window not less than 15. At the start of the original data sequencing reads, bases with a quality score lower than 3 are trimmed, and at the end of the sequencing reads, bases with a quality score lower than 3 are also trimmed. All other parameters without explicitly specified, the default software parameters are used. The quality control standards for ctDNA samples are as follows: $Q30 \geq 80\%$, sequencing depth before remove of deduplicate reads $\geq 15000X$, and a deduplication rate $\geq 2.5$. The BWA software is used to align the quality-controlled sequencing data to the human reference genome (version Hg19), thereby obtaining the effective alignment files (BAM files, containing effective alignment data) of healthy individual samples for subsequent analysis.

2. Acquiring the mutation frequencies of detected loci within the sequencing panel

Based on the BAM files, the sequencing depth of each locus and the number of reads supporting each of four possible nucleotide substitutions (A/C/T/G) within the panel range (Onco Sonar product, panel size approximately 0.3M) are counted. The single-nucleotide variant frequency of each of four nucleotides at each locus is calculated (the frequency of undetected single-nucleotide variant is recorded as 0). Specifically, the BAM file is used to generate a pileup-format file using mpileup command in the Samtools (v1.7, http://www.htslib.org/doc/samtools.html). Using a program (ctDNA-SNV-InDel pipeline, Genetron Health(Beijing) Co., Ltd.), the sequencing depth (i.e., the total number of sequencing reads at each locus) and the number of reads supporting mutation at each locus within the panel range are counted. The mutation frequency of single-nucleotide variant (SNV) at each locus is calculated by dividing the number of reads supporting SNV at each locus by the sequencing depth of that locus. Similarly, based on the BAM files, the sequencing depth (total number of sequencing reads at the locus) and the number of reads supporting insertion or deletion (InDel) of each locus within the panel range are counted. The mutation frequency of InDel at each locus is calculated by dividing the number of reads supporting InDel at each locus by the sequencing depth of that locus.

3. Screening characteristic groups and constructing a noise level model for single-nucleotide variant

3.1 Screening groups with identical SNV feature and determining the prior background noise level of single-nucleotide variant in each group

The Mappability file of the human reference genome is downloaded from the database developed and maintained by the University of California, Santa Cruz (hereinafter referred to as the UCSC database) (website: https://genome.ucsc.edu/cgi - bin/hgTables/hgsid = 1510554375_HANSVqcS3xxfWO2Ui7wYz8FFIlzA) (genome version: Hg19; 100bp reads length alignment version). This file indicates whether a certain genomic sequence position has a unique alignment or multiple alignments across the whole genome. Based on the reference genome file (version: Hg19), the tri-nucleotide sequence context (tri-nucleotide context) of each locus (i.e., the nucleotide sequence of a certain locus on the reference genome and a upstream and downstream nucleotide forming a three-nucleotide sequence) is extracted. The tri-nucleotide sequence context and the alignment feature (i.e., unique alignment or multiple alignments) are used as feature for subsequent model construction.

The tri-nucleotide sequences context and the alignment feature of each locus are combined for single-nucleotide variant grouping. Specifically, loci with the same tri-nucleotide sequence and the same alignments information are grouped together, and the loci within the group are those with the same single-nucleotide variant characteristics. According to different tri-nucleotide sequences and the alignments information, a maximum of 128 groups can be divided (each nucleotide at a locus has 4 possible forms (A/T/C/G), and the maximum number of three - nucleotide arrangements is $4^3 = 64$; the alignments information is divided into 2 cases: unique alignment and multiple alignments; the tri-nucleotide sequence context and alignment information can be divided into a maximum of $64 \times 2 = 128$ groups, and each group sharing the same identical locus feature). The loci within the panel sequencing range are grouped according to the above content (the tri-nucleotide sequence and the alignments information corresponding to each locus are unique, so each locus will only belong to a unique group). Based on the single-nucleotide variant frequencies of the loci obtained in Step 2, the average single-nucleotide variant frequency of all loci (loci with the same feature) in the group with identical SNV feature is calculated by taking the average of the single-nucleotide variant frequencies of all loci in the group (Formula 1). This average single-nucleotide variant frequency is used as the prior background noise level of single-nucleotide variant in the group:

$$\lambda_{tm} = \frac{\sum \mu_{ptm}}{n}$$

Formula 1.

In Formula 1: $\lambda_{tm}$ represents the average single-nucleotide variant frequency of all loci within the group with identical SNV feature, $\mu_{ptm}$ represents the single-nucleotide variant frequency of each locus within the group with identical SNV feature, and n represents the number of loci within the group with identical SNV feature.

3.2 Constructing a noise level model for single-nucleotide variant

By combining the group sharing the same SNV feature to which each locus belongs and the corresponding prior background noise level for single-nucleotide variant (the average single-nucleotide variant frequency of all loci within the group with identical SNV feature obtained in Step 3.1) with the single-nucleotide variant frequency detected at each locus in Step 2, a model is constructed to calculate the noise level of single-nucleotide variant at the current locus as follows:

$$\varepsilon_p = \omega_1 \lambda_{tm} + (1 - \omega_1)\mu_p$$

Formula 2

$$\omega_1 = \frac{\lambda_{tm}}{\lambda_{tm} + \mu_p}$$

Formula 3.

In Formulas 2 and 3: p represents a certain locus within the range of sequencing panel, such as chr7: 55249071; $\lambda_{tm}$ represents the average single-nucleotide variant frequency of all loci within the group with identical SNV feature to which locus p belongs; $\mu_p$ represents the single-nucleotide variant frequency of locus p; $\omega_1$ represents the weight; $\varepsilon_p$

represents the single-nucleotide variant noise level of locus p.

4. Screening insertion or deletion characteristic groups and constructing a noise level model for insertions or deletions mutations

4.1 Screening groups with identical InDel feature and determining the prior background noise level of insertions or deletions mutations in each group

The repeat sequence element data file (Repeats file, website: https://genome.ucsc.edu/cgi - bin/hgTables, genome version Hg19, select RepeatMasker) and the segmental duplication data file (Segmental Duplication file, website: https://genome.ucsc.edu/cgi - bin/hgTables, genome version Hg19, select Segmental Dups) of the human genome are downloaded from the UCSC database. The Repeats file provides information on whether a certain genomics locus belongs to a repeat sequence region and the specific type of the repeat region, which is divided into 8 different types: DNA, LINE, SINE, Low_complexity, Simple_repeats, LTR, NonRepeats, and Other (the DNA type represents DNA repeat elements; the LINE type represents long insert fragment repeat elements; the SINE type represents short insert fragment repeat elements; the Low complexity type represents repeat elements in low-complexity regions, such as G-rich and AT - rich regions; the Simple repeats type represents repeat elements in simple repeat regions, such as (TTAGGG)n and (GATATA)n; the LTR type represents long-fragment repeat region elements; the NonRepeats type represents loci located in non-repeat regions; the Other type represents other repeat elements, and detailed information on specific types can be referred to the UCSC database). The Segmental Duplication file provides information on whether a certain genomics locus belongs to a large-fragment repeat region (Segmental Duplication regions can be divided into non-large-fragment repeat regions and large-fragment repeat regions). The repeat sequence element information (Repeats information) and segmental duplication information (Segmental Duplication information) of the loci are used as features for subsequent construction of the noise level model for insertions or deletions mutations.

Loci with the same Repeats information (i.e., belonging to the same one of the 8 types, for example, all are DNA repeat elements) and the same Segmental Duplication information are grouped together, called groups with identical InDel feature, and the loci within the group are those with the same insertion or deletion characteristics. The Repeats information can be divided into 8 groups; the Segmental Duplication information can be divided into 2 groups. The combination of Repeats information and Segmental Duplication information can be divided into a maximum of $8 \times 2 = 16$ groups, and each group is called a group with identical insertion or deletion feature. The loci within the panel range are grouped (the Repeats information and Segmental Duplication information of each locus are unique, so each locus will only belong to a unique group). Based on the mutation frequencies of insertions or deletions mutations of the loci obtained in Step 2, the average mutation frequency is calculated by taking the average of the insertion or deletion frequencies of all loci in the group with identical insertion or deletion feature (Formula 4), which servers as the prior background noise level of insertions or deletions in the group:

$$\lambda_{rsl} = \frac{\sum \mu_{prsl}}{m} \qquad \text{Formula 4.}$$

In Formula 4: $\lambda_{rsl}$ represents the average insertion or deletion frequency of all loci within the group with identicalInDel feature, $\mu_{prsl}$ represents the insertion or deletion frequency of each locus within the group with identical InDel feature, and m represents the number of loci within the group with identical InDel feature.

4.2 Constructing a noise level model for insertions or deletions mutations

By combining the group sharing the same InDel feature to which each locus belongs and the corresponding prior background noise level for insertions or deletions (the average variant frequency of all loci in the group with identical InDel feature obtained in Step 3.1) with the insertion or deletion frequency detected at each locus in Step 2 using Formula 5 and 6, a model is constructed to calculate the noise level of insertions or deletions mutations at the current locus as follows:

$$\omega_2 = \frac{\lambda_{rsl}}{\lambda_{rsl}+\mu_{pl}} \qquad \text{Formula 5}$$

$$\varepsilon_{pl} = \omega_2\lambda_{rsl} + (1 - \omega_2)\,\mu_{pl} \qquad \text{Formula 6.}$$

In Formulas 5 and 6: p represents a certain locus within the range of sequencing panel, $\omega_2$ represents the weight, $\lambda_{rsl}$ represents the average insertion or deletion frequency of all loci within the group with identical InDel feature to which locus p belongs, $\mu_{pl}$ represents the insertion or deletion frequency at locus p, and $\varepsilon_{pl}$ represents the insertion or deletion noise level at locus p.

The noise level models for single-nucleotide variant at genomic loci and the noise level models for insertions or deletions at genomic loci together constitute the ctDNA NGS sequencing data noise level models.

Example 2: A process for identifying whether the mutations in ctDNA sequencing data are true mutations or sequencing noise.

For the samples to be tested, ctDNA sequencing data is obtained through processes such as DNA extraction, library construction, probe capture, and on-machine sequencing based on the same standard experimental procedure as in Example 1. After steps such as data pre-processing and alignment, for samples that pass quality control, the sequencing depth of the locus, the number of mutation-supporting reads, and the mutation frequency are counted to obtain the mutations of the ctDNA sequencing data of the sample to be tested. According to the noise level model constructed in Example 1, for loci with mutations in the sample to be tested, statistical testing methods are used to identify whether the mutations (single-nucleotide variants, insertion and deletion mutations) are true mutations or sequencing noise.

The ctDNA NGS sequencing data is subjected to quality control and alignment to the reference genome (reference genome version: Hg19) using the method in Example 1 to obtain BAM files. Based on the BAM files, at each locus, the sequencing depth of mutation, the number of mutation-supporting reads (the number of reads supporting the mutation) is counted, and the mutation frequency of the locus is calculated. Based on the locus information (i.e., the chromosome number and genomic position of the mutated locus) and the type of mutation (whether the mutation is a single-nucleotide variant or an insertion or deletion), the noise level of the locus obtained from the model constructed in Example 1 is matched (single-nucleotide variants are matched with single-nucleotide variant noise levels, and insertions or deletions are matched with insertion or deletion noise levels). According to the concept of statistical hypothesis testing, H0: The mutation at this locus is sequencing noise; H1: The mutation at this locus is a true mutation. Based on the sequencing depth of the mutation locus, the number of mutation-supporting reads, and the mutation noise level of the locus, the statistical significance level (P-value) of the locus is calculated (Formula 7 and Formula 8). If P-value < alpha (positive threshold), H0 should be rejected and H1 accepted, indicating that the detected mutation is a true mutation; otherwise, H0 is accepted, indicating that the detected mutation is sequencing noise.

$$\lambda_p = d_p \cdot \delta_p \qquad \text{Formula 7;}$$

$$P(k_p, \lambda_p) = 1 - \sum_{j=0}^{k_p} \frac{e^{-\lambda_p} \lambda_p^j}{j!} \qquad \text{Formula 8.}$$

In Formulas 7 and 8: p represents a certain locus within the range of sequencing panel, and $\delta_p$ is the noise level at locus p ( SNV noise level, InDel noise level); $d_p$ is the sequencing depth at locus p, $\lambda_p$ is the number of reads supporting noise mutation at locus p; $k_p$ is the number of reads supporting mutation (SNV, InDel) at locus p in the sample to be tested; j represents the value of the number of reads supporting mutation, $j \in (0, k_p)$; $P(k_p, \lambda_p)$ represents the statistical P-value when $k_p$ is greater than or equal to $\lambda_p$.

In this example, the positive threshold for mutations is 0.0001.

Example 3: Verification of mutations based on ctDNA sequencing data from tumor samples in the present disclosure.

The mutations determination methods established in Examples 1 and 2 of the present disclosure are compared with the prior art based on ctDNA sequencing data from tumor samples, with the evaluation metric being the number of detected known positive variants (i.e., single-nucleotide variants, insertions, or deletions). Ten previously clinically tested tumor samples (including 5 lung cancer samples and 5 colorectal cancer samples) collected by Genetron Health (Beijing) Co., Ltd. are selected. Following the Onco Sonar product experimental procedure, NGS sequencing data is obtained through processes such as DNA extraction, equal-proportion sample pooling, library construction, probe capture, and on-machine sequencing.

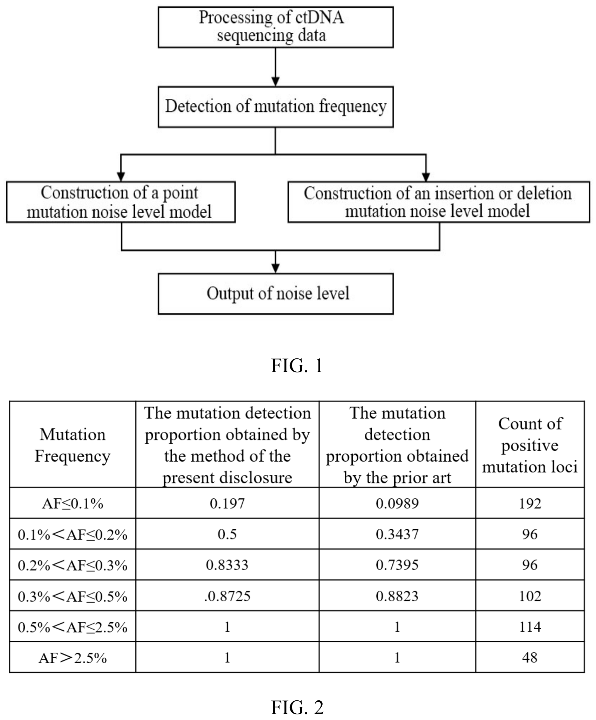

The prior art (i.e., using the samtools software's mpileup command to count the total depth, number of mutation-supporting reads, and mutation frequency of variant loci in ctDNA sequencing data. Criteria for determining true mutations: mutation frequency $\geq$ 0.1%, number of mutation-supporting reads $\geq$ 4; reference URL: http://www.htslib.org/doc/1.7/samtools.html) is used to separately analyze the sequencing data of the 10 samples (i.e., without the DNA equal-proportion sample pooling process). The detection results (see FIG. 2) show that there are a total of 648 positive mutation loci, among which 486 are low-frequency mutations (mutation frequency < 0.5%). Among the low-frequency mutations, there are 192 loci with a gene mutation frequency AF (Allele Frequency) $\leq$ 0.1%, 96 loci with 0.1 < AF $\leq$ 0.2%, 96 loci with 0.2 < AF $\leq$ 0.3%, and 102 loci with 0.3 < AF $\leq$ 0.5%.

The prior art and the method established in Example 2 are respectively used to analyze the sequencing data after sample pooling, and the mutation results are compared. By comparing the proportion of detected known positive mutation loci, it is found that the mutation detection method of the present disclosure has advantages in detecting low-frequency mutations (below 0.5%), with a performance improvement of approximately 26%.

Calculation method is shown in FIG. 2, for a certain frequency group, the performance improvement ratio of the current group is calculated by dividing the difference between the mutation detection proportion obtained by the method of the present disclosure and the mutation detection proportion obtained by the prior art. Detailed calculation steps: Performance improvement ratio for the AF $\leq$ 0.1% group: (0.197 - 0.0989) / 0.0989 = 99%; Performance improvement ratio for the 0.1 < AF $\leq$ 0.2% group: (0.5 - 0.3437) / 0.3437 = 45%; Performance improvement ratio for the 0.2 < AF $\leq$ 0.3% group: (0.8333 - 0.7395) / 0.7395 = 13%; Performance improvement ratio for the 0.3 < AF $\leq$ 0.5% group: (0.8725 - 0.8828) / 0.8823 = -1%; Performance improvement ratio for the 0.5 < AF $\leq$ 2.5% group: (1 - 1) / 1 = 0%; Performance improvement ratio for the AF > 2.5% group: (1 - 1) / 1 = 0%. Based on the performance improvement ratios of each frequency group mentioned above, the average value of the performance improvement ratios of the 6 frequency groups is calculated as the overall performance improvement ratio: (99% + 45% + 13% - 1% + 0% + 0%) / 6 = 26%.

In conclusion, the present disclosure calculates the statistical significance level P-value of a locus based on the sequencing depth of the locus in ctDNA sequencing data, the number of mutation-supporting reads, and the noise level of the locus, rather than relying on a uniformly defined criterion for positive mutations. It is particularly suitable for the detection of low-frequency mutations and will not lead to missed detections due to not reaching the threshold. The present disclosure does not require the provision of normal control samples, reducing cost investment and shortening the analysis cycle.

The present disclosure also provides a computer device, comprising a memory, a processor, and a computer program stored on the memory, wherein the processor executes the computer program to implement the following steps:

B1) Processing ctDNA sequencing data to obtain ctDNA sequencing data from healthy subjects, and aligning the data with the human reference genome after quality control to generate the alignment files;

B2) Performing detection of mutation frequency, based on the alignment files in B1), to obtain the sequencing depth, the number of reads supporting single-nucleotide variant , and the number of reads supporting insertion or deletion - at each locus in the ctDNA sequencing data. At each locus, calculate the single-nucleotide variant frequency based on the sequencing depth and the number of reads supporting single-nucleotide variant , and calculate the insertion or deletion frequency based on the sequencing depth and the number of reads supporting insertion or deletion ;

B3) Constructing a single-nucleotide variant noise level model to obtain the single-nucleotide variant noise level at each locus based on the grouping information of SNV feature at each locus and the single-nucleotide variant frequency at each locus, wherein the t grouping information of SNV feature at each locus includes the trinucleotide sequence context and the alignment feature;

B4) Constructing an insertion or deletion noise level model to obtain the insertion or deletion noise level at each locus based on the grouping information of InDel feature at each locus and the insertion or deletion frequency at each locus, wherein the grouping information of InDel feature at each locus includes the repetitive sequence element information and the segmental duplication information.

B5) Outputting the noise level of the ctDNA sequencing data, which includes the SNV noise level at each locus and the InDel noise level at each locus.

The construction of the single-nucleotide variant noise level model in B3) includes the following steps:

B3-1) Integrating and grouping single-nucleotide variant groups based on the trinucleotide sequence context and the alignment feature at each locus, to group loci with the same trinucleotide sequence context and the same alignment feature together, thereby forming groups with identical SNV feature;

B3-2) Acquiring prior background noise level of single-nucleotide variant to calculate the average single-nucleotide variant frequency of the group with identical SNV feature using Formula 1, based on the single-nucleotide variant frequency of all loci within the group with identical SNV feature and the group with identical SNV feature in B3-1):

$$\lambda_{tm} = \frac{\sum \mu_{ptm}}{n} \qquad \text{Formula 1,}$$

in Formula 1: $\lambda_{tm}$ represents the average single-nucleotide variant frequency of all loci within the group with identical SNV feature, $\mu_{ptm}$ represents the single-nucleotide variant frequency at each locus within the group with identical SNV feature in B3-1), and n represents the number of loci within the group with identical SNV feature in B3-1);

B3-3) Acquiring the single-nucleotide variant noise level at each locus using Formulas 2 and 3, based on the average single-nucleotide variant frequency of the group with identical SNV feature to which each locus belongs and the single-nucleotide variant frequency at each locus:

$$\varepsilon_p = \omega_1 \lambda_{tm} + (1 - \omega_1) \mu_p \qquad \text{Formula 2}$$

$$\omega_1 = \frac{\lambda_{tm}}{\lambda_{tm} + \mu_p} \qquad \text{Formula 3,}$$

in Formulas 2 and 3: p represents a certain locus within the range of sequencing Panel, $\lambda_{tm}$ represents the average single-nucleotide variant frequency of all loci within the group with identical SNV features to which locus p belongs, $\mu_p$ represents the single-nucleotide variant frequency at locus p, $\omega_1$ represents the weight, and $\varepsilon_p$ represents the single-nucleotide variant noise level at locus p.

The construction of the insertion or deletion noise level model in B4) includes the following steps:

B4-1) Integrating and grouping insertion or deletion groups based on the repetitive sequence element information and the segmental duplication information at each locus, to group loci with the same repetitive sequence element information and the same segmental duplication information together, thereby forming groups with identical InDel feature;

B4-2) Acquiring the prior background noise level of insertion or deletion to calculate the average insertion or deletion frequency of the group with identical InDel feature using Formula 4, based on the insertion or deletion frequency at each locus of the group with identical InDel feature and the group with identical InDel feature described in B4-1):

$$\lambda_{rsl} = \frac{\sum \mu_{prsl}}{m} \qquad \text{Formula 4,}$$

in Formula 4: $\lambda_{rsl}$ represents the average insertion or deletion frequency of all loci within the group with identical InDel feature, $\mu_{prsl}$ represents the insertion or deletion frequency at each locus within the group with identical InDel feature in B4-1), and m represents the number of loci within the group with identical InDel feature in B4-1);

B4-3) Acquiring the insertion or deletion noise level to at each locus using Formulas 5 and 6, based on the average insertion or deletion frequency of the group with identical InDel feature to which each locus belongs and the insertion or deletion frequency at each locus:

$$\omega_2 = \frac{\lambda_{rsl}}{\lambda_{rsl} + \mu_{pl}} \qquad \text{Formula 5}$$

$$\varepsilon_{pl} = \omega_2 \lambda_{rsl} + (1 - \omega_2) \mu_{pl} \qquad \text{Formula 6.}$$

In Formulas 5 and 6: p represents a certain locus within the range of sequencing Panel, $\omega_2$ represents the weight, $\lambda_{rsl}$ represents the average insertion or deletion frequency of all loci within the group with identical InDel feature to which locus p belongs, $\mu_{pl}$ represents the insertion or deletion frequency at locus p, and $\varepsilon_{pl}$ represents the insertion or deletion noise level at locus p.

The present disclosure also provides a computer device, comprising a memory, a processor, and a computer program stored on the memory, wherein the processor executes the computer program to implement the following steps:

D1) Acquiring ctDNA sequencing data from the sample to be tested, performing quality control and aligning the data with the human reference genome to generate alignment files;

D2) Performing mutation analysis of ctDNA sequencing data, based on the alignment files in D1), to obtain the sequencing depth, the number of reads supporting mutation, and the mutation frequency at each locus, thus obtaining the mutations in the ctDNA sequencing data from the sample to be tested;

D3) Performing statistical hypothesis testing analysis to determine whether the mutations are true mutations or sequencing noise using a statistical hypothesis testing method based on the mutations and the noise levels. The mutations are SNVs and/or InDels, and the noise level is the SNV noise level and/or the InDel noise level, which is obtained using the method described above.

The statistical hypothesis testing uses Formulas 7 and 8 to calculate the statistical significance level of the hypothesis test:

$$\lambda_p = d_p \cdot \delta_p \qquad \text{Formula 7}$$

$$P(k_p, \lambda_p) = 1 - \sum_{j=0}^{k_p} \frac{e^{-\lambda_p} \lambda_p^j}{j!} \qquad \text{Formula 8.}$$

In Formulas 7 and 8: p represents a certain locus within the range of sequencing Panel, $\delta_p$ is the noise level at locus p, $d_p$ is the sequencing depth at locus p, $\lambda_p$ is the expected number of reads supporting noise mutation observed at locus p, $k_p$ is the number of reads supporting mutation at locus p in the sample to be tested, j represents the value of the number of reads supporting mutation, $j \in (0, k_p)$, and $P(k_p, \lambda_p)$ represents the statistical P-value when $k_p$ is greater than or equal to $\lambda_p$.

The present disclosure has been described in detail above. For those skilled in the art, within the spirit and scope of the present disclosure and without the need for unnecessary experiments, the present disclosure can be implemented within a wide range of equivalent parameters, concentrations, and conditions. Although specific embodiments of the present disclosure have been given, it should be understood that further improvements can be made to the present disclosure. In summary, according to the principles of the present disclosure, this application intends to include any modifications, uses, or improvements to the present disclosure, including changes made using conventional techniques known in the art that deviate from the scope already disclosed in this application.

Cross-Reference to Related Applications

This application claims the priority of Chinese Patent Application (Application No. 202310929989.3) filed on July 27, 2023, the entire contents of which are hereby incorporated by reference into this document.

Industrial Applicability

The technical solution of the present disclosure can detect SNVs and InDels in single tumor sample ctDNA data without the need for data from matched normal control samples from tumor patients, which can reduce the investment in experimental and sequencing costs. The present disclosure can be applied to detect low-frequency mutations in tumor samples or to prepare products for detecting low-frequency mutations in tumor samples, thereby providing personalized medication guidance for tumor patients.

**Claims**

1. An apparatus for detecting the noise level in ctDNA sequencing data, wherein the apparatus comprises the following modules:

A1) a ctDNA sequencing data processing module: configured to obtain ctDNA sequencing data of healthy subjects, perform quality control on the data, and align the data with the human reference genome to generate alignment files.;

A2) a mutation frequency detection module: configured to obtain the sequencing depth at each locus, the number of reads supporting single-nucleotide variant at each locus, and the number of reads supporting insertion or deletion at each locus in the ctDNA sequencing data based on the alignment files. For each locus, the module calculates: (i) the single-nucleotide variant frequency based on the sequencing depth and the number of reads supporting single-nucleotide variant, and (ii) the insertion or deletion frequency based on the sequencing depth and the number of reads supporting insertion or deletion;

A3) a single-nucleotide variant noise level model construction module: configured to obtain the single-nucleotide variant noise level at each locus based on the grouping information of single-nucleotide variant feature at each locus and the single-nucleotide variant frequency at each locus; wherein the grouping information of single-nucleotide variant feature at each locus includes the trinucleotide sequence context and the alignment feature at each locus;

A4) an insertion or deletion noise level model construction module: configured to obtain the insertion or deletion noise level at each locus based on the grouping information of insertion or deletion feature at each locus and the insertion or deletion frequency at each locus; wherein the grouping information of insertion or deletion at each locus includes the repetitive sequence element information and the segmental duplication information at each locus;

A5) a noise level output module: configured to output the noise levels of the ctDNA sequencing data, which include the SNV noise level at each locus and/or the InDel noise level at each locus.

2. The apparatus according to claim 1, wherein the single-nucleotide variant noise level model construction module in a3) comprises the following modules:

A3-1) a single-nucleotide variant characteristic integration and grouping module: configured to group loci that share the same trinucleotide sequence context and the same alignment feature together, thereby forming groups with identical SNV feature;

A3-2) a single-nucleotide variant prior background noise level acquisition module: configured to calculate the average single-nucleotide variant frequency of each group with identical SNV feature using formula 1, based on the mutation frequencies of all loci within the group with identical SNV feature and the group with identical SNV feature described in A3-1):

$$\lambda_{tm} = \frac{\sum \mu_{ptm}}{n} \qquad \text{formula 1};$$

in formula 1, $\lambda_{tm}$ represents the average single-nucleotide variant frequency of all loci within the group with identical SNV feature, $\mu_{ptm}$ represents the single-nucleotide variant frequency at each locus within the group with identical SNV feature described in A3-1), and n represents the number of loci within the group with identical SNV feature described in A3-1);

A3-3) a single-nucleotide variant noise level acquisition module: configured to obtain the single-nucleotide variant noise level at each locus using formulas 2 and 3, based on the average single-nucleotide variant frequency of the group with identical SNV feature to which each locus belongs and the single-nucleotide variant frequency at each locus:

$$\varepsilon_p = \omega_1 \lambda_{tm} + (1 - \omega_1) \mu_p \quad \text{formula 2};$$

$$\omega_1 = \frac{\lambda_{tm}}{\lambda_{tm} + \mu_p} \qquad \text{formula 3};$$

in formulas 2 and 3, p represents a certain locus within the range of the sequencing Panel; $\lambda_{tm}$ represents the average single-nucleotide variant frequency of all loci within the group with identical SNV feature to which locus p belongs; $\mu_p$ represents the single-nucleotide variant frequency at locus p; $\omega_1$ represents weight; $\varepsilon_p$ represents the single-nucleotide variant noise level at locus p.

3. The apparatus according to claim 1, wherein the insertion or deletion mutation noise level model construction module in A4) comprises the following modules:

A4-1) an insertion or deletion characteristic integration and grouping module: configured to group loci that share the same repetitive sequence element information and the same segmental duplication information together, thereby forming groups with identical InDel feature ;

A4-2) an insertion or deletion mutation prior background noise level acquisition module: configured to calculate the average insertion or deletion frequency of each group with identical InDel feature using formula 4, based on the insertion or deletion mutation frequency of all loci within the group with identical InDel feature and the group with identical InDel feature described in A4-1):

$$\lambda_{rsl} = \frac{\sum \mu_{prsl}}{m} \qquad \text{formula 4;}$$

in formula 4, $\lambda_{rsl}$ represents the average insertion or deletion frequency of all loci within the group with identical InDel feature, $\mu_{prsl}$ represents the insertion or deletion mutation frequency at each locus within the group with identical InDel feature described in A4-1), and m represents the number of loci within the group with identical InDel feature described in A4-1);

A4-3) an insertion or deletion mutation noise level acquisition module: configured to obtain the insertion or deletion noise level at each locus using formulas 5 and 6, based on the average insertion or deletion mutation frequency of the group with identical InDel feature to which each locus belongs and the insertion or deletion frequency at each locus:

$$\omega_2 = \frac{\lambda_{rsl}}{\lambda_{rsl} + \mu_{pl}} \qquad \text{formula 5;}$$

$$\varepsilon_{pl} = \omega_2 \lambda_{rsl} + (1 - \omega_2)\mu_{pl} \quad \text{formula 6;}$$

in formulas 5 and 6, p represents a certain locus within the range of the sequencing panel; $\omega_2$ represents the weight; $\lambda_{rsl}$ represents the average insertion or deletion mutation frequency of all loci within the group with identical InDel feature to which locus p belongs; $\mu_{pl}$ represents the insertion or deletion mutation frequency at locus p; $\varepsilon_{pl}$ represents the insertion or deletion mutation noise level at locus p.

4. An apparatus for identifying whether the mutations in ctDNA sequencing data are true mutations or sequencing noise, comprising the following modules:

C1) a ctDNA sequencing data quality control and alignment module: configured to obtain ctDNA sequencing data of the sample to be tested, perform quality control, and align the data with the human reference genome to generate alignment files;

C2) a ctDNA sequencing data mutation analysis module: configured to obtain the sequencing depth, the number of reads supporting mutation, and the mutation frequency at each locus based on the alignment files, thereby obtaining mutations in the ctDNA sequencing data of the sample to be tested;

C3) a statistical testing module: configured to determine whether the mutations are true mutations or sequencing noise using statistical hypothesis testing methods based on the mutations and noise levels; the mutations are SNVs and/or InDels; the noise levels are SNV noise levels and/or InDel noise levels, and the noise levels are obtained using the apparatus described in any one of claims 1-3.

5. The apparatus according to claim 4, wherein the statistical hypothesis test calculates the statistical significance level of the hypothesis test by using formulas 7 and 8:

$$\lambda_p = d_p \cdot \delta_p \qquad \text{formula 7;}$$

$$P(k_p, \lambda_p) = 1 - \sum_{j=0}^{k_p} \frac{e^{-\lambda_p} \lambda_p^j}{j!} \qquad \text{formula 8;}$$

in formulas 7 and 8, p represents a certain locus within range of sequencing panel; $\delta_p$ is the noise level at locus p; $d_p$ is the sequencing depth at locus p; $\lambda_p$ is the expected number of reads supporting noise mutation observed at locus p; $k_p$ is the number of reads supporting mutation at locus p in a sample to be tested; j represents the value of the number of reads supporting mutation, $j \in (0, k_p)$; $P(k_p, \lambda_p)$ represents the statistical P-value when $k_p$ is greater than or equal to $\lambda_p$.

6. A method for detecting the noise levels in ctDNA sequencing data, comprising the following steps:

B1) processing ctDNA sequencing data to obtain ctDNA sequencing data of healthy subjects, and align the data with the human reference genome after quality control to generate the alignment files ;

B2) performing detection of mutation frequency: based on the alignment files, to obtain the sequencing depth, the number of reads supporting single-nucleotide variant, and the number of reads supporting insertion or deletion at each locus in the ctDNA sequencing data. At each locus, calculate the single-nucleotide variant frequency based on the sequencing depth and the number of reads supporting single-nucleotide variant, and calculate the insertion or deletion mutation frequency based on the sequencing depth and the number of reads supporting insertion or deletion;

B3) constructing single-nucleotide variant noise level model to obtain the single-nucleotide variant noise level at each locus based on the grouping information of SNV feature at each locus and the single-nucleotide variant frequency at each locus, wherein the grouping information of SNV feature at each locus includes the trinucleotide sequence context and of the alignment feature;

B4) constructing insertion or deletion mutation noise level model to obtain the insertion or deletion noise level at each locus based on the grouping information of InDel feature at each locus and the insertion or deletion frequency at each locus; wherein the grouping information of InDel feature at each locus includes the repetitive sequence element information and the segmental duplication information;

B5) outputting the noise level in the ctDNA sequencing data, which include the SNV noise level at each locus and/or the InDel noise level at each locus.

7. The method according to claim 6, wherein the constructing single-nucleotide variant noise level model in B3) comprises the following steps:

B3-1) integrating and grouping single-nucleotide variant groups based on the trinucleotide sequence context and alignment feature at each locus, to group loci with the same trinucleotide sequence context and the same alignment feature together, thereby forming groups with identical SNV feature;

B3-2) acquiring the prior background noise level of single-nucleotide variant to calculate the average single-nucleotide variant frequency of the group with identical SNV feature using formula 1, based on the single-nucleotide variant frequency of all loci within the group with identical SNV feature and the group with identical SNV feature described in B3-1):

$$\lambda_{tm} = \frac{\sum \mu_{ptm}}{n} \qquad \text{formula 1;}$$

in formula 1, $\lambda_{tm}$ represents the average single-nucleotide variant frequency of all loci within the group with identical SNV feature, $\mu_{ptm}$ represents the single-nucleotide variant frequency at each locus within the group with identical SNV feature described in B3-1), and n represents the number of loci within the group with identical SNV feature described in B3-1);

B3-3) acquiring the single-nucleotide variant noise level at each locus using formulas 2 and 3, based on the average single-nucleotide variant frequency of the group with identical SNV feature to which each locus belongs and the single-nucleotide variant frequency at each locus:

$$\varepsilon_p = \omega_1 \lambda_{tm} + (1 - \omega_1) \mu_p \qquad \text{formula 2;}$$

$$\omega_1 = \frac{\lambda_{tm}}{\lambda_{tm} + \mu_p} \qquad \text{formula 3;}$$

in formulas 2 and 3, p represents a certain locus within the range of sequencing panel; $\lambda_{tm}$ represents the average single-nucleotide variant frequency of all loci within the group with identical SNV feature to which locus p belongs; $\mu_p$ represents the single-nucleotide variant frequency at locus p; $\omega_1$ represents weight; $\varepsilon_p$ represents the single-nucleotide variant noise level at locus p.

8. The method according to claim 6 or 7, wherein the constructing insertion or deletion noise level model in B4) comprises the following steps:

B4-1) integrating and grouping insertion or deletion groups based on the repetitive sequence element information and the segmental duplication information at each locus, to group loci with the same repetitive sequence element information and the same segmental duplication information together, thereby forming groups with identical InDel feature;

B4-2) acquiring the prior background noise level of insertion or deletion to calculate the average insertion or deletion frequency of the group with identical InDel feature using formula 4, based on the insertion or deletion frequency at each locus of the group with identical InDel feature and the group with identical InDel feature described in B4-1):

$$\lambda_{rsl} = \frac{\sum \mu_{prsl}}{m} \qquad \text{formula 4;}$$

in formula 4, $\lambda_{rsl}$ represents the average insertion or deletion frequency of all loci within the group with identical InDel feature, $\mu_{prsl}$ represents the insertion or deletion frequency at each locus within the group with identical InDel feature described in B4-1), and m represents the number of loci within the group with identical InDel feature described in B4-1);

B4-3) acquiring the insertion or deletion noise level at each locus using formulas 5 and 6, based on the average insertion or deletion frequency of the group with identical InDel feature to which each locus belongs and the insertion or deletion frequency at each locus:

$$\omega_2 = \frac{\lambda_{rsl}}{\lambda_{rsl} + \mu_{pl}} \qquad \text{formula 5;}$$

$$\varepsilon_{pl} = \omega_2 \lambda_{rsl} + (1 - \omega_2) \mu_{pl} \qquad \text{formula 6;}$$

in formulas 5 and 6, p represents a certain locus within the range of sequencing panel; $\omega_2$ represents the weight; $\lambda_{rsl}$ represents the average insertion or deletion frequency of all loci within the group with identical InDel feature to which locus p belongs; $\mu_{pl}$ represents the insertion or deletion frequency at locus p; $\varepsilon_{pl}$ represents the insertion or deletion noise level at locus p.

9. A method for identifying whether the mutations in ctDNA sequencing data are true mutations or sequencing noise, comprising the following steps:

D1) acquiring the ctDNA sequencing data of the samples to be tested, performing quality control and aligning the data with the human reference genome to generate alignment files;

D2) performing mutation analysis on ctDNA sequencing data to obtain the sequencing depth, the number of reads supporting mutation, and the mutation frequency at each locus based on the alignment files, thereby obtaining the mutations in the ctDNA sequencing data of the sample to be tested;

D3) performing statistical testing analysis to determine whether the mutations are true mutations or sequencing noise using a statistical hypothesis testing method based on the mutations and the noise levels; the mutations are SNVs and/or InDels; the noise levels are SNV noise levels and/or InDel noise levels, and the noise levels are obtained using the method described in any one of claims 6-8.

10. The method according to claim 9, wherein statistical hypothesis test calculates a statistical significance level of the hypothesis test by using formulas 7 and 8:

$$\lambda_p = d_p \cdot \delta_p \qquad \text{Formula 7;}$$

$$P(k_p, \lambda_p) = 1 - \sum_{j=0}^{k_p} \frac{e^{-\lambda_p} \lambda_p^j}{j!} \qquad \text{Formula 8;}$$

in formulas 7 and 8, p represents a certain locus within the range of sequencing panel; $\delta_p$ is the noise level at locus p; $d_p$ is the sequencing depth at locus p; $\lambda_p$ is the expected number of reads supporting noise mutation observed at locus p;

$k_p$ is the number of reads supporting mutation at locus p in a sample to be tested; j represents the value of the number of reads supporting mutation, $j \in (0, k_p)$; $P(k_p, \lambda_p)$ represents the statistical P-value when $k_p$ is greater than or equal to $\lambda_p$.

11. A computer-readable storage medium storing a computer program, wherein the computer-readable storage medium is a computer-readable storage medium for detecting a noise level in ctDNA sequencing data; the computer program enables a computer to execute steps of the method described in any one of claims 6-8.

12. A computer-readable storage medium storing a computer program, wherein the computer-readable storage medium is a computer-readable storage medium for identifying whether the mutations in ctDNA sequencing data are true mutations or sequencing noise; the computer program enables a computer to execute steps of the method described in claim 9 or 10.

13. Any one of the following uses of the apparatus described in any one of claims 1-5 or the method described in any one of claims 6-10:

    E1) use in preparing products for screening or auxiliary screening of cancer patients;
    E2) use in development or preparing products for personalized cancer therapy.

14. Any one of the following uses of the apparatus described in claim 4 or 5:

    E1) use in preparing products for screening or auxiliary screening of cancer patients;
    E2) use in development or preparing products for personalized cancer therapy.

15. Any one of the following uses of the method described in any one of claims 6-8:

    E1) use in preparing products for screening or auxiliary screening of cancer patients;
    E2) use in development or preparing products for personalized cancer therapy.

16. Any one of the following uses of the method described in claim 9 or 10:

    E1) use in preparing products for screening or auxiliary screening of cancer patients;
    E2) use in development or preparing products for personalized cancer therapy.

FIG. 1

| Mutation Frequency | The mutation detection proportion obtained by the method of the present disclosure | The mutation detection proportion obtained by the prior art | Count of positive mutation loci |
|---|---|---|---|
| AF≤0.1% | 0.197 | 0.0989 | 192 |
| 0.1%＜AF≤0.2% | 0.5 | 0.3437 | 96 |
| 0.2%＜AF≤0.3% | 0.8333 | 0.7395 | 96 |
| 0.3%＜AF≤0.5% | .0.8725 | 0.8823 | 102 |
| 0.5%＜AF≤2.5% | 1 | 1 | 114 |
| AF＞2.5% | 1 | 1 | 48 |

FIG. 2

# INTERNATIONAL SEARCH REPORT

International application No.

**PCT/CN2024/107680**

| A. | CLASSIFICATION OF SUBJECT MATTER |
|---|---|

G16B20/50(2019.01)i; G16B20/30(2019.01)i; G16B40/10(2019.01)i

According to International Patent Classification (IPC) or to both national classification and IPC

| B. | FIELDS SEARCHED |
|---|---|

Minimum documentation searched (classification system followed by classification symbols)

IPC:G16B

Documentation searched other than minimum documentation to the extent that such documents are included in the fields searched

Electronic data base consulted during the international search (name of data base and, where practicable, search terms used)

CJFD, CNABS, CNTXT, ENTXT, ENTXTC, VEN, BING, WEB OF SCIENCE, PUBMED: 北京泛生子基因科技有限公司, 循环肿瘤DNA, ctDNA, cfDNA, 噪音, 噪声, 背景, 基线, 比对, 参考基因组, 健康+, 频率, 突变频率, SNV, InDel, 测序深度, 支持, 三核苷酸, 重复, noise, noise level, background, align, reference geome, frequency, depth, supporting, reads, duplicate, healthy individual

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 116356001 A (JIANGSU SIMCERE DIAGNOSTICS CO., LTD.) 30 June 2023 (2023-06-30)<br>description, paragraphs 2-3, 10-55, and 120, and figure 1 | 1, 4-6, 9-16 |
| PX | CN 116646007 A (GENETRON HEALTH (BEIJING) CO, LTD.) 25 August 2023 (2023-08-25)<br>claims 1-12, and embodiment 1 | 1-16 |
| PX | CHE, Dongxue et al. "BayVarC: an Ultra-Sensitive CtDNA Variant Caller Using Bayesian Approach"<br>*BioRxiv, https://www.biorxiv.org/content/10.1101/2024.02.03.578772v1.full.pdf,*<br>07 February 2024 (2024-02-07),<br>pages 1-24 | 1, 4-6, 9-16 |
| A | CN 106845153 A (ANNOROAD GENE TECHNOLOGY (BEIJING) CO., LTD.) 13 June 2017 (2017-06-13)<br>entire document | 1-16 |

☑ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **15 October 2024** | **22 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/107680** |

| C. | DOCUMENTS CONSIDERED TO BE RELEVANT | |
| --- | --- | --- |
| Category* | Citation of document, with indication, where appropriate, of the relevant passages | Relevant to claim No. |
| A | CN 114141310 A (GUANGZHOU BURNING ROCK DX CO., LTD.) 04 March 2022 (2022-03-04)<br>    entire document | 1-16 |
| A | CN 114596918 A (SUZHOU JIYINJIA BIOMEDICAL ENGINEERING CO., LTD. et al.) 07 June 2022 (2022-06-07)<br>    entire document | 1-16 |
| A | US 2019108311 A1 (GRAIL, INC.) 11 April 2019 (2019-04-11)<br>    entire document | 1-16 |
| A | 杨飘等 (YANG, Piao et al.). "MinerVa--一种用于实体瘤微小残留病灶 检测的高性能检测算法 (Non-official translation: MinerVa--A High-Performance Detection Algorithm for Micro-Residual Lesion Detection in Solid Tumors)"<br>生物医学工程学杂志 *(Journal of Biomedical Engineering)*,<br>Vol. 40, No. 2, 30 April 2023 (2023-04-30),<br>    pages 313-319 | 1-16 |

Form PCT/ISA/210 (second sheet) (July 2022)

EP 4 738 368 A1

**INTERNATIONAL SEARCH REPORT**
**Information on patent family members**

International application No.

**PCT/CN2024/107680**

| Patent document cited in search report | | | Publication date (day/month/year) | Patent family member(s) | | | Publication date (day/month/year) |
|---|---|---|---|---|---|---|---|
| CN | 116356001 | A | 30 June 2023 | None | | | |
| CN | 116646007 | A | 25 August 2023 | None | | | |
| CN | 106845153 | A | 13 June 2017 | None | | | |
| CN | 114141310 | A | 04 March 2022 | None | | | |
| CN | 114596918 | A | 07 June 2022 | None | | | |
| US | 2019108311 | A1 | 11 April 2019 | WO | 2019071219 | A1 | 11 April 2019 |
| | | | | TW | 201928797 | A | 16 July 2019 |
| | | | | TWI | 781230 | B | 21 October 2022 |
| | | | | EP | 3676846 | A1 | 08 July 2020 |

Form PCT/ISA/210 (patent family annex) (July 2022)

24

**EP 4 738 368 A1**

**Patent documents cited in the description**

- CN 202310929989